(19) Europäisches Patentamt
European Patent Office
Office européen des brevets

(11) Veröffentlichungsnummer : **0 020 817**
**B1**

(12) # EUROPÄISCHE PATENTSCHRIFT

(45) Veröffentlichungstag der Patentschrift : **19.05.82**

(51) Int. Cl.³ : **C 07 D251/20**

(21) Anmeldenummer : **79105360.6**

(22) Anmeldetag : **24.12.79**

(54) **Verfahren zur Herstellung von Pyrenverbindungen.**

(30) Priorität : **08.06.79 DE 2923337**

(43) Veröffentlichungstag der Anmeldung : **07.01.81 (Patentblatt 81/01)**

(45) Bekanntmachung des Hinweises auf die Patenterteilung : **19.05.82 Patentblatt 82/20**

(84) Benannte Vertragsstaaten : **CH DE FR GB**

(56) Entgegenhaltungen :
**GB - A - 985 484**
**GB - A - 1 221 930**

(73) Patentinhaber : **BAYER AG**
**Zentralbereich Patente, Marken und Lizenzen**
**D-5090 Leverkusen 1, Bayerwerk (DE)**

(72) Erfinder : **Harnisch, Horst, Dr.**
**Heinenbusch 4**
**D-5203 Much (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 020 817 B1

# Verfahren zur Herstellung von Pyrenverbindungen

Gegenstand der vorliegenden Erfindung ist ein Verfahren zur Herstellung von Verbindungen der Formel

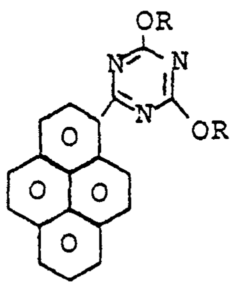

(I)

worin R für einen gegebenenfalls substituierten Alkyl-, Alkenyl-, Cycloalkyl-, Aralkyl- oder Arylrest steht.

Solche Verbindungen haben als optische Aufheller für Textilmaterialien wie Polyester und Kunststoffe technische Bedeutung erlangt. Sie wurden bislang in der Weise hergestellt, daß man in eine Mischung aus Pyren und Cyanurchlorid in einem geeigneten Lösungsmittel Aluminiumchlorid einträgt, das Reaktionsprodukt nach der Methanolyse oder Hydrolyse isoliert, durch Ausrühren mit eiskalter Salzsäure von Aluminiumsalzen befreit, nochmals isoliert, mit Methanol wäscht, trocknet und in einem weiteren Reaktionsschritt die Chloratome durch RO-Reste ersetzt (US-PS 2 232 871, GB-PS 985 484 und GB-PS 1 221 930).

Diese Herstellungsmethode weist jedoch eine Reihe von Nachteilen auf. Insbesondere lassen die Ausbeuten und die Reinheit der Verfahrensprodukte zu wünschen übrig. Es hat daher an Versuchen, die Herstellung zu verbessern, nicht gefehlt. So wird für den speziellen Fall, daß R = Methyl ist, in der GB-PS 1 144 002 die Umsetzung von 2-Chlor-4,6-dimethoxy-triazin, das in einem gesonderten Reaktionsschritt aus Cyanurchlorid herzustellen ist, mit Pyren in Gegenwart von Aluminiumchlorid empfohlen. Jedoch liefert auch dieses Verfahren — laut Angaben der Patentschrift — nur eine 55 %ige Ausbeute.

Aufgabe der vorliegenden Erfindung war es daher, ein rationelles Verfahren zu entwickeln, daß zu reineren Produkten führt.

Erfindungsgemäß wird nun diese Aufgabe dadurch gelöst, daß man durch Umsetzung eines Aluminiumhalogenids mit mindestens der äquivalenten Menge Cyanurchlorid in einem inerten Lösungsmittel einen Komplex herstellt, diesen auf Pyren einwirken läßt und das Reaktionsprodukt — vorteilhaft ohne Zwischenisolierung — mit mindestens der für die austauschbaren Halogenatome äquivalenten Menge einer Verbindung der Formel

$$RO—M \qquad (II)$$

worin

R die oben angegebene Bedeutung besitzt und M für ein Alkalimetall, vorzugsweise Natrium oder Kalium

steht, umsetzt.

Man erhält nach diesem Verfahren hohe Ausbeuten, in der Regel von 80-90 % der Theorie bezogen auf Pyren. Die Verfahrensprodukte fallen in so hoher Reinheit an, daß im allgemeinen eine zusätzliche Reinigung nicht erforderlich ist.

Das bei der Einwirkung von Pyren auf den farblosen Cyanurchlorid-Aluminiumhalogenid-Komplex gebildete Reaktionsprodukt ist ein blauer 1-(2,4-Dichlor-1,3,5-triazin-6-yl)-pyrenhydrochlorid-Aluminiumhalogenid-Komplex, der sich überraschenderweise leichter und vollständiger mit Verbindungen der Formel II umsetzt als das durch nachträgliche Methanolyse oder Hydrolyse und Anschließende Entfernung der Aluminiumsalze bisher daraus hergestellte 1-(2,4-Dichlor-1,3,5-triazin-6-yl)-pyren selbst.

Daß das erfindungsgemäße Verfahren so glatt verläuft und so ausgezeichnete Ausbeuten und Reinheit der Verfahrensprodukte liefert, ist auch deshalb überraschend, weil die Acylierung von Kohlenwasserstoffen in Gegenwart von Friedel-Crafts-Katalysatoren zumeist nicht quantitativ ablaufen und die Aluminiumsalze im allgemeinen bei Folgeumsetzungen stören, so daß die Acylierungsprodukte in der Regel hydrolysiert, isoliert und gereinigt werden müssen, bevor man sie weiteren Reaktionen zuführen kann.

Weiterhin ist überraschend, daß man auf den sonst für Friedel-Crafts-Acylierungen von Kohlenwasserstoffen erforderlichen größeren Überschuß an Katalysator (in der Regel 50 Mol%) — und somit im vorliegenden Falle auch an Cyanurchlorid — weitgehend verzichten kann. Dies hat den entscheidenden Vorteil, daß in der Folgereaktion mit Alkoholat bzw. Phenolat ein allzu großer Salzballast und die damit verbundene erforderliche Vergrößerung des Reaktionsvolumen vermieden werden kann.

Das neue Verfahren eignet sich besonders zur Herstellung solcher Verbindungen der Formel I, worin

R für gegebenenfalls durch Hydroxy, $C_1$-$C_4$-Alkoxy oder Phenoxy substituiertes $C_1$-$C_5$-Alkyl, $C_2$-$C_5$-Alkenyl, Cyclohexyl oder einen gegebenenfalls durch Methyl oder Chlor substituierten Phenyl-$C_1$-$C_3$-alkyl- oder Phenylrest steht.

Besonders bevorzugt sind $C_1$-$C_4$-Alkylreste R, insbesondere Methyl.

Besonders geeignet ist das neue Verfahren auch zur Hestellung von Gemischen aus Verbindungen der Formeln

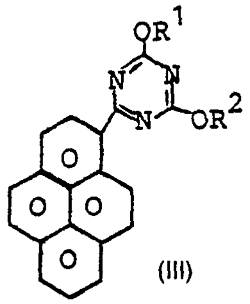
(III)

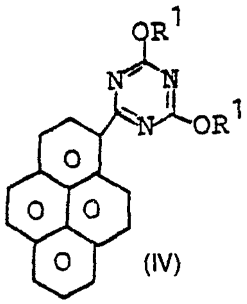
(IV)

und

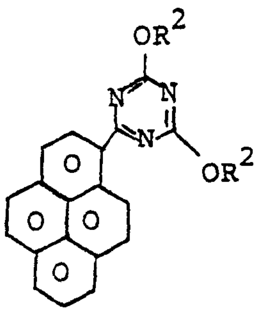
(V)

die ebenfalls wertvolle Weißtöner-Eigenschaften, beispielsweise zum optischen Aufhellen von Polyestermaterialien, besitzen.

Man erhält diese Verbindungen dadurch, daß man in dem erfindungsgemäßen Verfahren anstelle einer einheitlichen Verbindung der Formel RO—M, worin R die oben angegebene Bedeutung besitzt, eine äquivalente mischung aus Komponenten der Formel $R^1OM$ und $R^2OM$,
worin
$R^1$ und $R^2$ voneinander verschieden sind und jeweils eine der für R angegebenen Bedeutungen besitzen,
einsetzt.

Bei der praktischen Durchführung des Verfahrens geht man zweckmäßigerweise so vor, daß man in einem inerten Lösungsmittel bei 0-25 °C, vorzugsweise 10-20 °C ein Aluminiumhalogenid mit mindestens der äquivalenten Menge Cyanurchlorid mehrere Stunden verrührt, auf den erhaltenen farblosen Cyanurchlorid-Aluminiumhalogenid-Komplex bei 0-25 °C, vorzugsweise 10-20 °C unter Kühlung Pyren einwirken läßt, wobei sich der tiefblaue Aluminiumhalogenid-Komplex ausbildet, diesen — vorteilhaft ohne Zwischenisolierung — bei 0-40 °C, vorzugsweise 0-25 °C, unter starker Kühlung mit mindestens der für die insgesamt anwesenden austauschbaren Halogenatome äquivalenten Menge der Verdindung der Formel RO—M, worin R die oben angegebene Bedeutung besitzt, vermischt, danach — vorteilhalterweise in einer Inertgasatmosphäre — die Reaktionsmischung kurz auf 50-80 °C, vorzugsweise 60-75 °C erhitzt, wobei man ein über 5 Stunden ausgedehntes Erhitzen auf 60 °C und höher zweckmäßig vermeidet, die Reaktionsmischung abkühlen läßt, das Verfahrensprodukt isoliert, zunächst mit einem polaren, mit Wasser mischbaren organischen Lösungsmittel wie Methanol oder Ethanol und schließlich mit Wasser wäscht.

Das Endprodukt fällt, wie oben erwähnt, in der Regel in so hoher Reinheit an, daß keine zusätzlichen Reinigungsoperationen erforderlich sind.

Geeignete inerte Lösungsmittel sind beispielsweise Halogen- oder Nitrokohlenwasserstoffe, wie Dichlormethan, Trichlormethan, Tetrachlormethan, 1,2-Dichlorethan, Hexachlorethan, Chlorbenzol, o-Dichlorbenzol, Chlortoluole, Dichlortuole, Trichlorbenzol, Nitromethan, Nitropropan und nitrobenzol. Bevorzugt sind die aromatischen Chlor- und Nitrokohlenwasserstoffe, insbesondere o-Dichlorbenzol, Trichlorbenzol und Nitrobenzol.

Als Aluminiumhalogenide kommen Aluminiumbromid und insbesondere Aluminiumchlorid in Betracht.

Die Verbindungen der Formel RO—M können vorteilhaft als Lösung in der betreffenden ROH-Verbindung, sofern diese flüssig ist, beispielsweise als 30 %ig methanolische Natriummethylatlösung, oder mit gleichem Erfolg auch als Mischung aus Verbindungen der Formel ROH und Alkalihydroxiden der Formel MOH eingesetzt werden.

Pro Mol Pyren setzt man zweckmäßig nur 1-1,1 Mol Aluminiumhalogenid sowie 1-1,2 Mol Cyanurchlorid ein. Wichtig ist, daß während der Pyrenzugabe möglichst kein freies Aluminiumhalogenid mehr vorliegt.

Für die in der Reaktionsmischung insgesamt anwesenden austauschbaren Halogenatome sind pro Mol Pyren mindestens 6 Äquivalente an Verbindung der Formel RO—M erforderlich, 3 Äquivalente für Cyanurchlorid und 3 Äquivalente für das Aluminiumhalogenid. Zweckmäßig verwendet man 6,5-9,5 Äquivalente.

Beispiel 1

Zu 2 000 ml wasserfreiem o-Dichlorbenzol werden bei 15-18 °C nacheinander 243,6 g Cyanurchlorid (1,32 Mol) und 168 g wasserfreies Aluminiumchlorid-Pulver (1,26 Mol) zugegeben und 8 Std. in diesem Temperaturbereich verrührt. Anschließend setzt man unter Kühlung bei derselben Temperatur 242,4 g pulv. Pyren (1,2 Mol) hinzu (30-60 Min.). Hierbei ist auf eine gute Durchmischung der Komponenten zu achten und örtliche Überhitzung zu vermeiden. Man rührt die tief blauviolette Suspension 2 Std. bei 15-18 °C nach. Nun fügt man unter starker Kühlung bei 10-18 °C 1 800 g 30 %ige methanolische Natriummethylatlösung (10 Mol) hinzu (1,5 Std.), rührt die Mischung noch 6 Std. < 20° und anschließend 30 Min. bei 70 °C (Rückfluß) und Läßt sie in sich abkühlen. Bei Raumtemperatur wird der kristalline Niederschlag abgesaugt, zweimal mit insgesamt 1 l Methanol gewaschen, gut abgepreßt, in mehrerem Portionen mit insgesamt 16 l Wasser gewaschen und bei 90 °C im

Vakuum getrocknet. Ausbeute : 344 g (84,5 % der Theorie) 1-(2,4-Dimethoxy-1,3,5-triazin-6-yl)-pyren vom Schmelzpunkt 199 °C. Eine zusätzliche Reinigung ist nicht erforderlich.

Setzt man anstelle von o-Dichlorbenzol ein gleiches Volumen wasserfreies Nitrobenzol ein, so erhält man 352 g (86 % der Theorie) eines ebenso reinen 1-(2,4-Dimethoxy-1,3,5-triazin-6-yl)-pyrens.

Beispiel 2

Zu 800 ml wasserfreiem o-Dichlorbenzol werden bei 15-18 °C nacheinander 250 g Cyanurchlorid (1,36 Mol) und 168 g wasserfreies Aluminiumchlorid-Pulver (1,26 Mol) zugegeben und 12 Std. im angegebenen Temperaturbereich verrührt. Anschließend setzt man unter Kühlung ebenfalls bei 15-18 °C — anfangs Tropfen für Tropfen — eine Lösung von 242,4 g Pyren (1,2 Mol) in 1 l wasserfreiem o-Dichlorbenzol hinzu und rührt die tief blauviolette Suspension 2 Std. bei 15-18 °C nach.

In einem 2. Reaktionsgefäß legt man 1 760 g 30 %ige Natriummethylatlösung vor. Mit Hilfe eines möglichst tief in die gerührte Suspension des $AlCl_3$-Komplexes eintauchenden Glasrohres zieht man diese mit schwachem Unterdruck tropfenweise in die stark gekühlte Natriummethylatlösung unter Rühren bei 10-15 °C ein (3 Std.). Anschließend spült man das 1. Reaktionsgefäß mit 200 ml wasserfreim o-Dichlorbenzol aus und zieht die so erhaltene Suspension in gleicher Weise bei 10-15 °C in die Methylatlösung ein. Man läßt die Mischung noch 6 Std. unter 20 °C nachrühren und erhitzt sie dann 1 Std. unter $N_2$ zum Sieden. Nach dem Abkühlen auf 20 °C und 3-stündigem Verrühren bei dieser Temperatur wird der kristalline Niederschlag abgesaugt, mit 1 l Methanol gewaschen, gut abgepreßt und mit reichlich Wasser gewaschen.

Ausbeute (feucht) : 630 g = 348 g
Trockengewicht = 85 % der Theorie.
Schmelzpunkt 199,5-200 °C.

Beispiel 3

Man verrührt in 1 l wasserfreiem o-Dichlorbenzol 121,8 g Cyanurchlorid (0,66 Mol) und 84 g wasserfreies Aluminiumchlorid-Pulver (0,63 Mol) < 20 °C. Nach 4 Std. setzt man unter Kühlung < 20 °C eine Suspension von 121,2 g Pyren (0,6 Mol) in 350 ml o-Dichlorbenzol zu.

Man rührt die Mischung noch 2 Stunden < 20° nach. Nun fügt man bei 20-25 °C 331 g 2-Phenoxyethanol und unter starker Kühlung bei 10-20 °C nach und nach 307,4 g Kaliumhydroxid-Pulver (tech., 88 %ig, 4,83 Mol) hinzu und läßt die Mischung 5 Std. < 20° nachreagieren. Der kristalline Niederschlag wird abgesaugt, mit 500 ml Methanol und mit 4 l Wasser gewaschen und bei 80 °C im Vakuum getrocknet. Ausbeute : 269 g (81 % der Theorie) 1-(2,4-Di-β-phenoxyethoxy-1,3,5-triazin-6-yl)-pyren vom Schmelzpunkt 185-186 °C. Eine zusätzliche Reinigung ist nicht erforderlich.

Beispiel 4

Man verfährt nach den Angaben des Beispiels 1, setzt aber anstelle der Natriumethylatlösung eine Mischung aus 870 g 30 %ig. methanolischer Natriummethylatlösung und 1 636 g 20 %ig. ethanolischer Natriumethylatlösung ein. Ausbeute 360 g einer Mischung aus 1-(2,4-Dimethoxy-1,3,5,triazin-6-yl)-pyren, 1-(2,4-Diethoxy-1,3,5-triazin-6-yl)-pyren und 1-(2-Methoxy-4-ethoxy-1,3,5-triazin-6-yl)-pyren vom Schmelzpunkt 151-157 °C.

Beispiel 5

Verfährt man nach den Angaben des Beispiels 3, setzt jedoch anstelle von 2-Phenoxyethanol äquivalente Mengen der nachstehend aufgeführten Alkohole ein, so erhält man die entsprechenden Oxytriazinylpyrene in den in Klammern angegebenen Ausbeuten :

| | |
|---|---|
| Allylalkohol | (81 %) |
| Benzylalkohol | (87 %) |
| n-Butanol | (88 %) |
| Methoxyethanol | (86 %) |
| Hydroxyethanol | (83 %) |
| Cyclohexanol | (78 %) |
| γ-Phenyl-n-propanol | (82 %) |

**Ansprüche**

1. Verfahren zur Herstellung von Pyrenverbindungen der Formel

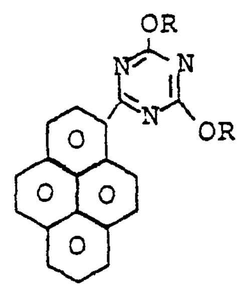

worin

R für einen gegebenenfalls substituierten Alkyl-, Alkenyl-, Cycloalkyl-, Aralkyl- oder Arylrest steht, durch Umsetzung Cyanurchlorid mit Pyren in Gegenwart eines Aluminiumhalogenids und weitere Umsetzung des Zwischenprodukts mit Alkalialkoholat oder -phenolat, dadurch gekennzeichnet, daß man durch Umsetzung eines Aluminiumhalogenids mit mindestens der äquivalenten Menge Cyanurchlorid in einem inerten Lösungsmittel einen Komplex herstellt, diesen auf Pyren einwirken läßt und das Reaktionsprodukt mit mindestens der für die austauschbaren Halogen atome äquivalenten Menge einer Verbindung der Formel

RO—M

worin
R die oben angegebene Bedeutung besitzt und
M für ein Alkalimetall steht,
umsetzt.

2. Verfahren nach Anspruch 1, dadurch gekennzeichnet, daß man weder den Komplex aus Cyanurchlorid und dem Aluminiumhalogenid noch das daraus durch Einwirkung von Pyren erhaltene Reaktionsprodukt zwischenisoliert.

3. Verfahren nach Anspruch 1, dadurch gekennzeichnet, daß man als aluminiumhalogenid $AlCl_3$ verwendet.

4. Verfahren nach Anspruch 1, dadurch gekennzeichnet, daß man zur Herstellung des Cyanurchlorid-Aluminiumhalogenid-Komplexes pro Mol Pyren 1-1,1 Mol Aluminiumhalogenid und 1-1,2 Mol Cyanurchlorid einsetzt.

5. Verfahren nach Anspruch 1, dadurch gekennzeichnet, daß man die Umsetzung des Aluminiumhalogenids mit Cyanurchlorid und die Folgereaktion mit Pyren bei 0-25 °C durchführt, das Vermischen der Verbindung RO—M mit dem Reaktionsprodukt aus dem Cyanurchlorid-Aluminiumhalogenid-Komplex und Pyren bei 0-40 °C vornimmt und die Austauschreaktion durch Erwärmen auf 50-80 °C vervollständigt.

6. Verfahren nach Anspruch 1, dadurch gekennzeichnet, daß man zum Austausch von Halogen gegen die Reste der Formel RO- pro Mol Pyren 6,5-9,5 Mol Verbindung der Formel RO—M einsetzt.

7. Verfahren nach Anspruch 1, dadurch gekennzeichnet, daß man die Suspension des Reaktionsproduktes aus dem Cyanurchlorid-Aluminiumhalogenid-Komplex und Pyren im inerten organischen Lösungsmittel in die gekühlte Lösung von RO—M in RO—H einträgt.

8. Verfahren nach Anspruch 1, dadurch gekennzeichnet, daß man Verbindungen der Formel RO—M in Form von Mischungen aus ROH und MOH einsetzt.

9. Verfahren nach Anspruch 1, dadurch gekennzeichnet, daß man anstelle einer einheitlichen Verbindung der Formel RO—M eine äquivalente Mischung aus Komponenten der Formeln

$R^1O—M$ und $R^2O—M$

worin
$R^1$ und $R^2$ voneinander verschieden sind und jeweils eine der für R angegebenen Bedeutungen besitzen,
einsetzt.

10. Verfahren zur Herstellung eines 1-(2,4-Dichlor-1,3,5-triazin-6-yl)-pyren-hydrochlorid-Aluminiumhalogenid-Komplexes durch Umsetzung von Cyanurchlorid, Pyren und eines Aluminiumhalogenids, dadurch gekennzeichnet, daß man ein Aluminiumhalogenid mit mindestens der äquivalenten Menge Cyanurchlorid in einem inerten Lösungsmittel zu einem Komplex umsetzt und auf diesen Pyren einwirken läßt.

**Claims**

1. Process for the preparation of pyrene compounds of the formula

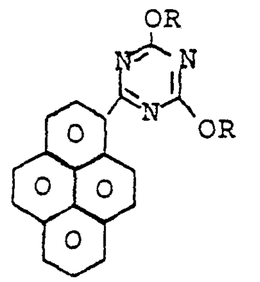

wherein
R represents an optionally substituted alkyl, alkenyl, cycloalkyl, aralkyl or aryl radical,
by reaction of cyanuric chloride with pyrene in the presence of an aluminium halide and by further reaction of the intermediate product with an alkali metal alcoholate or phenolate, characterised in that a complex is prepared by reacting an aluminium halide with at least an equivalent amount of cyanuric chloride in an inert solvent, this complex is allowed to act on pyrene and the product is reacted with a compound of the formula

RO—M

wherein
R has the abovementioned meaning and
M represents an alkali metal,
in an amount which is at least equivalent for the replaceable halogen atoms.

2. Process according to Claim 1, characterised in that neither the complex of cyanuric chloride and the aluminium halide nor the reaction product obtained therefrom by the action of pyrene is intermediately isolated.

3. Process according to Claim 1, characterised in that $AlCl_3$ is used as the aluminium halide.

4. Process according to Claim 1, characterised in that, per mol or pyrene, 1-1.1 mols of aluminium halide and 1-1.2 mols of cyanuric chloride are used for the preparation of the cyanuric chloride-aluminium halide complex.

5. Process according to Claim 1, characterised in that the reaction of the aluminium halide with cyanuric chloride and the subsequent reaction with pyrene are carried out at 0-25 °C, the compound RO—M is mixed with the reaction product of the cyanuric chloride-aluminium halide complex and pyrene at 0-40 °C and the replacement reaction is brought to completion by warming the mixture to 50-80 °C.

6. Process according to Claim 1, characterised in that, per mol of pyrene, 6.5-9.5 mols of compound of the formula RO—M are used for replacing halogen by the radicals of the formula RO.

7. Process according to Claim 1, characterised in that the suspension of the reaction product of the cyanuric chloride-aluminium halide complex and pyrene in the inert organic solvent is introduced into a cooled solution of RO—M in RO—H.

8. Process according to Claim 1, characterised in that compounds of the formula RO—M are used in the form of mixtures of ROH and MOH.

9. Process according to Claim 1, characterised in that, instead of a single compound of the formula RO—M, an equivalent mixture of components of the formulae

$$R^1O—M \quad \text{and} \quad R^2O—M$$

wherein

$R^1$ and $R^2$ are different and each have one of the meanings given for R,

is used.

10. Process for the preparation of a 1-(2,4-dichloro-1,3,5-triazin-6-yl)-pyrene hydrochloride-aluminium halide complex by reaction of cyanuric chloride, pyrene and an aluminium halide, characterised in that an aluminium halide is reacted with at least an equivalent amount of cyanuric chloride in an inert solvent to form a complex and pyrene is allowed to act on this complex.

**Revendications**

1. Procédé pour la préparation de composés de pyrène de formule générale

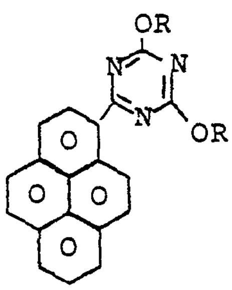

dans laquelle

R représente un reste alkyle, alcényle, cycloalkyle, aralkyle ou aryle éventuellement substitué,

par réaction du chlorure de cyanuryle avec le pyrène en présence d'un halogénure d'aluminium et réaction ultérieure du produit intermédiaire avec un alcoolate ou phénolate alcalin, caractérisé en ce que l'on prépare un complexe par réaction d'un halogénure d'aluminium avec au moins la quantité équivalente de chlorure de cyanuryle dans un solvant inerte, on fait réagir celui-ci sur le pyrène et on fait réagir le produit de réaction avec au moins la quantité équivalente pour les atomes d'halogène échangeables d'un composé de formule

$$RO—M$$

dans laquelle

R a la signification indiquée ci-dessus et

M représente un métal alcalin.

2. Procédé selon la revendication 1, caractérisé en ce que l'on n'isole ni le complexe de chlorure de cyanuryle et de l'halogénure d'aluminium ni le produit de réaction obtenu à partir de celui-ci par l'action du pyrène.

3. Procédé selon la revendication 1, caractérisé en ce que l'on utilise $AlCl_3$ comme halogénure d'aluminium.

4. Procédé selon la revendication 1, caractérisé en ce que l'on utilise pour la préparation du complexe chlorure de cyanuryle-halogénure d'aluminium 1-1,1 mole d'halogénure d'aluminium et 1-1,2 mole de chlorure de cyanuryle par mole de pyrène.

5. Procédé selon la revendication 1, caractérisé en ce que l'on effectue la réaction de l'halogénure d'aluminium avec le chlorure de cyanuryle et la réaction suivante avec le pyrène à 0-25 °C, on mélange le composé RO—M avec le produit de réaction du complexe chlorure de cyanuryle-halogénure d'aluminium et du pyrène à 0-40 °C et on complète la réaction d'échange par chauffage à 50-80 °C.

6. Procédé selon la revendication 1, caractérisé en ce que l'on utilise pour l'échange de l'halogène avec les restes de formule RO- 6,5-9,5 moles du composé de formule RO—M par mole de pyrène.

7. Procédé selon la revendication 1, caractérisé en ce que l'on ajoute dans la solution refroidie de RO—M dans RO—H la suspension du produit de réaction du complexe chlorure de cyanuryle-halogénure d'aluminium et du pyrène dans un solvant organique inerte.

8. Procédé selon la revendication 1, caractérisé en ce que l'on utilise les composés de formule RO—M sous forme de mélanges de RO—H et MO—H.

9. Procédé selon la revendication 1, caractérisé en ce que l'on utilise au lieu d'un composé unique de formule RO—M un mélange équivalent de composants de formules

$$R^1O—M \quad \text{et} \quad R^2O—M$$

où

$R_1$ et $R_2$ sont différents et possèdent chacun une des significations indiquées pour R.

10. Procédé pour la préparation d'un complexe chlorhydrate de 1-(2,4-dichloro-1,3,5-triazine-6-yl)-pyrène-halogénure d'aluminium par réaction du chlorure de cyanuryle, du pyrène et d'un halogénure d'aluminium, caractérisé en ce que l'on fait réagir un halogénure d'aluminium avec au moins la quantité équivalente de chlorure de cyanuryle dans un solvant inerte en un complexe et on fait agir le pyrène sur celui-ci.